(19) Europäisches Patentamt
European Patent Office
Office européen des brevets

(11) Numéro de publication: **0 551 510 B1**

# (12) FASCICULE DE BREVET EUROPEEN

(45) Date de publication du fascicule du brevet: **27.09.95**

(51) Int. Cl.$^6$: **A61K 7/42**

(21) Numéro de dépôt: **92918453.9**

(22) Date de dépôt: **05.08.92**

(86) Numéro de dépôt internationale :
**PCT/FR92/00774**

(87) Numéro de publication internationale :
**WO 93/02659 (18.02.93 93/05)**

## (54) COMPOSITION COSMETIQUE FILTRANTE PHOTOSTABLE CONTENANT UN FILTRE UV-A ET UN 4-METHOXY BENZYLIDENE CYANOACETATE.

(30) Priorité: **07.08.91 FR 9110061**

(43) Date de publication de la demande:
**21.07.93 Bulletin 93/29**

(45) Mention de la délivrance du brevet:
**27.09.95 Bulletin 95/39**

(84) Etats contractants désignés:
**DE FR GB IT**

(56) Documents cités:
**EP-A- 0 005 182**
**FR-A- 2 440 933**

**PATENT ABSTRACTS OF JAPAN vol. 11, no. 53 (C-404)(2500)**

(73) Titulaire: **L'OREAL**
**14, rue Royale**
**F-75008 Paris (FR)**

(72) Inventeur: **FORESTIER, Serge**
**16, allée Ferdinand-Buisson**
**F-77410 Claye-Souilly (FR)**
Inventeur: **DEFLANDRE, André**
**Route de Manon**
**F-60560 Orry-la-Ville (FR)**

(74) Mandataire: **Casalonga, Axel**
**BUREAU D.A. CASALONGA - JOSSE**
**Morassistrasse 8**
**D-80469 München (DE)**

**EP 0 551 510 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à une composition cosmétique photostable destinée à protéger la peau contre les rayonnements UV, contenant un filtre UV-A et un 4-méthoxy benzylidène cyanoacétate, à son utilisation pour la protection de la peau contre les rayons UV et un procédé de stabilisation du filtre UV-A par un 4-méthoxy benzylidène cyanoacétate.

On sait que les rayons UV-A, de longueurs d'onde comprises entre 320 et 400 nm, provoquant le brunissement de la peau, sont susceptibles d'induire une altération de celle-ci notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques. Il est donc souhaitable de filtrer le rayonnement UV-A.

Le brevet français n° 2 440 933 décrit le 4-(1,1-diméthyléthyl) 4'-méthoxy dibenzoylméthane à titre de filtre UV-A. Il est proposé d'associer ce filtre UV-A particulier, vendu sous la dénomination "PARSOL 1789" par la Société GIVAUDAN, à différents filtres UV-B dans le but d'absorber l'ensemble des rayonnements UV de longueurs d'onde comprises entre 280 et 380 nm.

Malheureusement, lorsque ce filtre UV-A est utilisé seul ou en association avec des filtres UV-B, il ne possède pas une stabilité photochimique satisfaisante pour garantir une protection constante de la peau durant une exposition solaire prolongée, ce qui nécessite des applications répétées à intervalles réguliers et rapprochés si l'on veut obtenir une protection efficace de la peau contre l'ensemble des rayons UV.

La demanderesse a découvert qu'en associant le 4-(1,1 - diméthyléthyl)4'-méthoxydibenzoylméthane à un filtre UV-A du type 4-méthoxy benzylidène cyanoacétate de formule :

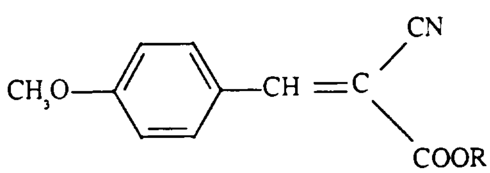

(I)

dans des proportions et dans un rapport molaire bien définis, on obtenait d'une manière surprenante, une stabilité photochimique du 4-(1,1-diméthyléthyl) 4'-méthoxydibenzoylméthane. On obtient ainsi une association stabilisée protégeant l'épiderme humain contre les rayons UV de longueurs d'onde comprises entre 320 et 380 nm. Cette association est particulièrement intéressante dans des produits de soin de la peau à usage quotidien dans le but de retarder son vieillissement.

On peut obtenir des compositions anti-solaires photostables filtrant l'ensemble des rayons UV de longueurs comprises entre 280 et 380 nm, en ajoutant, à l'association précitée des filtres UV-A, des filtres UV-B.

Dans la formule générale (I) le substituant R représente un radical alkyle, linéaire ou ramifié, comportant 6 à 12 atomes de carbone.

On peut citer par exemple, les radicaux n-hexyle, n-octyle, n-décyle, n-dodécyle, iso-octyle, iso-nonyle et iso-décyle.

Parmi les composés de formule (I) on préfère plus particulièrement :

- le 4-méthoxy benzylidène cyanoacétate de n-hexyle,
- le 4-méthoxy benzylidène cyanoacétate d'iso-nonyle.

Les composés de formule (I) ainsi que leur procédé de préparation sont décrits dans la demande EP 0005182.

De par leur caractère lipophile, les filtres utilisés se répartissent uniformément dans les supports cosmétiques classiques contenant au moins une phase grasse ou se présentant sous forme de dispersions aqueuses de vésicules lipidiques et peuvent ainsi être appliqués sur la peau pour constituer une film protecteur efficace.

La présente invention a pour objet une composition cosmétique photostable, protégeant la peau contre le rayonnement UV de longueurs d'onde comprises entre 320 et 380 nm, comprenant dans un support cosmétiquement acceptable, de 0,5 à 5% en poids de 4-(1,1-diméthyléthyl)4'-méthoxydibenzoylméthane et au moins 0,5% en poids de composé de formule (I), le rapport en mole du composé de formule (I) au 4-(1,1-diméthyléthyl)4'-méthoxydibenzoylméthane étant supérieur ou égal à 0,8, et de préférence supérieur

ou égal à 1,2.

La limite supérieure de ce rapport molaire est déterminée par la solubilité de ces filtres dans la phase grasse utilisée dans la composition ou dans la phase lipidique présente dans la dispersion vésiculaire.

La présente invention a aussi pour objet un procédé de protection de l'épiderme humain contre les effets des rayons UV de longueurs d'onde comprises entre 320 et 380 nm consistant à appliquer sur la peau une quantité efficace d'une composition cosmétique telle que définie ci-dessus.

Un autre objet de la présente invention est constitué par un procédé de stabilisation du 4-(1,1-diméthyléthyl)4'-méthoxy dibenzoylméthane vis-à-vis du rayonnement UV à l'aide d'un composé de formule (I), procédé dans lequel on utilise au moins 0,5% en poids de composé de formule (I) pour stabiliser de 0,5 à 5% en poids de 4-(1,1-diméthyléthyl)4'-méthoxydibenzoylméthane, le rapport en mole du composé de formule (I) au 4-(1,1-diméthyléthyl)4'-méthoxydibenzoylméthane étant égal ou supérieur à 0,8.

Les compositions cosmétiques selon l'invention peuvent se présenter sous forme de lotions huileuses ou oléoalcooliques, sous forme de gels gras ou oléoalcooliques, de bâtonnets solides, d'émulsions telles qu'une crème ou un lait, ou de dispersions vésiculaires de lipides amphiphiles ioniques ou non ioniques; elles peuvent être conditionnées en aérosol.

Comme solvant de solubilisation, on peut utiliser une huile ou une cire, un monoalcool ou un polyol inférieur ou leur mélange. Les monoalcools ou polyols particulièrement préférés sont l'éthanol, l'isopropanol, le propylèneglycol et la glycérine.

La composition cosmétique selon l'invention destinée à protéger l'épiderme humain contre les rayons ultraviolets peut contenir des adjuvants cosmétiques usuels dans ce type de composition tels que des épaississants, des adoucissants, des humectants, des tensio-actifs, des conservateurs, des anti-mousses, des huiles, des cires, de la lanoline, des parfums, des propulseurs, des colorants et/ou des pigments ayant pour fonction de colorer la composition elle-même ou la peau, ou tout autre ingrédient habituellement utilisé en cosmétique.

Une forme de réalisation de l'invention est une émulsion sous forme de crème ou de lait comprenant, en plus du composé de formule (I) associé au 4-(1,1-diméthyléthyl) 4'-méthoxydibenzoylméthane, des alcools gras, des esters d'acides gras et notamment des triglycérides d'acides gras, des acides gras, de la lanoline, des huiles, des cires naturelles ou synthétiques et des émulsionnants, en présence d'eau.

Une autre forme de réalisation est constituée de lotions huileuses à base d'esters d'acides gras, d'huiles et/ou de cires naturelles ou synthétiques ou de lotions oléoalcooliques à base d'huiles, de cires ou d'esters d'acides gras tels que les triglycérides d'acides gras et d'alcools inférieurs tels que l'éthanol ou de glycols, tel que le propylèneglycol et/ou de polyols comme la glycérine.

Les gels oléoalcooliques comprennent une huile ou une cire, un alcool ou un polyol inférieur comme l'éthanol, le propylèneglycol ou la glycérine et un épaississant tel que la silice.

Les bâtonnets solides sont constitués de corps gras comme les cires et huiles naturelles ou synthétiques, les alcools gras, les esters d'acides gras et la lanoline.

Les dispersions vésiculaires de lipides amphiphiles ioniques ou non-ioniques sont préparées selon des procédés connus comme par exemple, en faisant gonfler les lipides dans une solution aqueuse pour former des sphérules dispersées dans le milieu aqueux comme décrit dans l'article BANGHAN, STANDISH et WATKINS, J. Mol. Biol., 13, 238 (1965) ou dans les brevets FR 2 315 991 et 2 416 008 de la demanderesse. On trouvera la description des divers modes de préparation dans "Les liposomes en biologie cellulaire et pharmacologie". Edition INSERM/John Libbery Eurotext, 1987, pages 6 à 18.

Lorsque les compositions se présentent sous forme d'émulsion ou de dispersion vésiculaire, la phase aqueuse peut contenir en plus des filtres UV hydrosolubles tels que l'acide benzène 1,4-[di(3-méthylidène 10-camphosulfonique)], l'acide 2-phénylbenzimidazole 5-sulfonique ou l'acide 2-hydroxy 4-méthoxybenzophénone 5-sulfonique, ces acides étant salifiés ou non.

Dans le cas d'une composition conditionnée en aérosol, on utilise des propulseurs classiques tels que les alcanes, les fluoroalcanes et les chlorofluoroalcanes.

Les compositions selon l'invention peuvent également comporter des nanopigments d'oxyde métallique dispersés dans la phase grasse et/ou dans la phase aqueuse.

Un autre objet de la présente invention est constitué par une composition cosmétique photostable, antisolaire filtrant les rayons UV de longueurs d'onde comprises entre 280 et 380nm, caractérisée par le fait qu'elle contient en plus de l'association de filtres UV-A telle que définie ci-dessus, des filtres UV-B.

La présente invention concerne également un procédé de protection de l'épiderme humain contre les effets des rayons UV de longueurs d'onde comprises entre 280 et 380 nm consistant à appliquer une quantité efficace d'une composition contenant des filtres UVB et l'association de 4-(1,1-diméthyléthyl) 4'-méthoxy dibenzoylméthane et de composés de formule (I).

A titre de filtre UV-B, on peut citer par exemple, le benzylidène camphre et ses dérivés tel que le p-méthylbenzylidène camphre; les β,β-diphénylacrylates; les α-cyano-β,β-diphénylacrylates d'alkyle comme l'α-cyano β,β-diphénylacrylate de 2-éthylhexyle; les dialkylbenzalmalonates; les esters d'acide salicylique; les esters de l'acide p-amino benzoïque et leurs dérivés; les dérivés de benzophénone tels que le 2-hydroxy-4-méthoxy benzophénone et la 2,2'-dihydroxy 4'-méthoxy benzophénone.

Ces filtres UV-B sont présents dans des concentrations ne dépassant pas 10% en poids du poids total de la composition et de préférence comprises entre 0,5 et 8% en poids.

Les compositions anti-solaires de l'invention peuvent se présenter sous les mêmes formes que les compositions pour la protection de l'épiderme humain à usage quotidien.

Les exemples qui suivent servent à illustrer l'invention sans pour autant présenter un caractère limitatif.

EXEMPLES 1 à 4

| ***CONSTITUANTS*** *(en g)* | *Ex.* **1** | *Ex.* **2** | *Ex.* **3** | *Ex.* **4** |
|---|---|---|---|---|
| 4-(1,1-diméthyléthyl) 4'-méthoxy dibenzoylméthane | 1,5 | 1,5 | 1,5 | 1,75 |
| 4-méthoxy benzylidène cyano acétate de n-hexyle | 4,5 | - | 2,0 | 8,0 |
| 4-méthoxy benzylidène cyano acétate d'iso-nonyle | - | 4,5 | - | - |
| Mélange d'alcool cétylstéarylique et d'alcool cétylstéarylique oxyéthylény à 33 moles d'OE (1) | 7,0 | 7,0 | 7,0 | 7,0 |
| Mélange de stéarates de mono, di et triglycérol | 2,0 | 2,0 | 2,0 | 2,0 |
| Triglycérides d'acides gras en $C_8$-$C_{12}$ | 30,0 | 30,0 | 30,0 | 30,0 |
| Polydiméthylsiloxane | 1,5 | 1,5 | 1,5 | 1,5 |
| Alcool cétylique | 1,5 | 1,5 | 1,5 | 1,5 |
| Eau distillée QSP | 100 | 100 | 100 | 100 |

(1) Vendu sous la dénomination de "SINNOWAX 10" par la Société HENKEL

Les émulsions des exemples 1 à 4 sont préparées selon les techniques classiques en dissolvant les filtres dans la phase grasse contenant les émulsionnants, en chauffant cette phase grasse vers 80-85 °C et en ajoutant, sous vive agitation, l'eau préalablement chauffée vers 80 °C. Ces émulsions constituent des crèmes de jour pour protéger l'épiderme humain.

## EXEMPLE 5

| | |
|---|---|
| - Alcool cétylique polyglycérolé (3 moles) | 3,8 g |
| - Cholestérol | 3,8 g |
| - Sel monosodique du glutamate de formule :<br>$HOOC-CH_2-CH_2-CH-COONa$<br>$\qquad\qquad\qquad\quad$ \| <br>$\qquad\qquad\qquad NH-COR$<br>dans laquelle R est un mélange de radicaux alkényle et/ou alkyle hydrogénés en $C_{14}$-$C_{22}$ dérivé des acides gras du suif, vendu sous la dénomination commerciale "ACYLGLUTAMATE HS 11" par la société AJINOMOTO | 0,4 g |
| - Glycérine | 2,0 g |
| - Acide benzène 1,4-di(3-méthylidène-10-camphosulfonique) en solution aqueuse à 35% MA | 1,0 g MA |
| - Triéthanolamine | 0,6 g |
| - Benzoate d'alcools en $C_{12}$-$C_{15}$ vendu sous la dénomination "FINSOLV TN" par la société WITCO | 8,0 g |
| - Cyclotétradiméthylsiloxane | 5,0 g |
| - 4-(1,1-diméthyléthyl)4'-méthoxydibenzoylméthane | 2,0 g |
| - 4-méthoxybenzylidène cyanoacétate de n-hexyle | 4,0 g |
| - Hydroxyéthyl cellulose modifiée par une chaîne cétyle vendue sous la dénomination "NATROSOL PLUS GRADE 330 CS" par la société AQUALON | 0,5 g |
| - Conservateurs, agent séquestrant qs | |
| - Eau qsp | 100 g |

EXEMPLE 6

| | |
|---|---|
| - Mélange (80/20) d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Mono et distéarate de glycérol (40/50) | 2,0 g |
| - Alcool cétylique | 1,5 g |
| - Huile de silicone DC 200-350 cst de DOW CORNING | 1.5 g |
| - Huile de vaseline | 15,0 g |
| - 4-(1,1-diméthyléthyl)4'-méthoxydibenzoylméthane | 1,0 g |
| - 4-méthoxybenzylidène cyanoacétate de n-hexyle | 3,0 g |
| - Oxyde de titane enrobé d'alumine et de stéarate d'aluminium vendu sous la dénomination "MICRO TITANIUM DIOXIDE MT 100T" par la société TAYCA | 2,0 g |
| - Glycérine | 20,0 g |
| - Conservateurs, agent séquestrant, parfum qs | |
| - Eau qsp | 100 g |

## Revendications

1. Composition cosmétique filtrante, photostable, pour la protection de l'épiderme humain contre les rayons ultraviolets de longueurs d'onde comprises entre 320 et 380 nm, caractérisée par le fait qu'elle comprend dans un support cosmétiquement acceptable, de 0,5 à 5% en poids de 4-(1,1-diméthyléthyl) 4'-méthoxy dibenzoylméthane et au moins 0.5% en poids d'un 4-méthoxybenzylidène cyano acétate de formule :

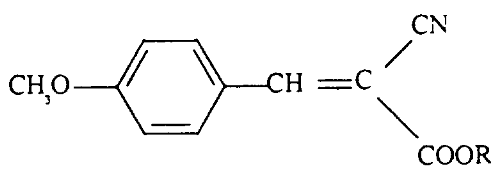

(I)

où R désigne un radical alkyle, linéaire ou ramifié, ayant 6 à 12 atomes de carbone; le rapport molaire du composé de formule (I) au 4-(1,1-diméthyléthyl) 4'-méthoxy dibenzoylméthane étant supérieur ou égal à 0,8.

2. Composition selon la revendication 1, caractérisée par le fait que le composé de formule (I) est choisi parmi le 4-méthoxybenzylidè-ne cyanoacétate de n-hexyle et le 4-méthoxy benzylidène cyano acétate d'iso-nonyle.

3. Composition selon la revendication 1 ou 2, caractérisée par le fait qu'elle se présente sous forme de lotion huileuse ou oléoalcoolique, sous forme de gel gras ou oléoalcoolique, de bâtonnet solide, d'émulsion, de dispersion vésiculaire de lipides amphiphiles ioniques ou non-ioniques ou est conditionnée en aérosol.

4. Composition selon l'une quelconque des revendications 1 à 3, caractérisée par le fait qu'elle contient en outre des adjuvants cosmétiques choisis parmi les épaississants, adoucissants, humectants, tensioactifs, conservateurs, anti-mousses, parfums, huiles, cires, mono-alcools et polyols inférieurs, propulseurs, colorants et pigments.

5. Composition selon l'une quelconque des revendications 1 à 4, caractérisée par le fait qu'elle constitue une émulsion sous forme de crème ou de lait comprenant à titre de support cosmétique, des alcools gras, des esters d'acides gras et notamment des triglycérides d'acides gras, des acides gras, de la lanoline, des huiles et des cires naturelles ou synthétiques et des émulsionnants, en présence d'eau.

**6.** Composition selon l'une quelconque des revendications 1 à 4, caractérisée par le fait qu'elle constitue une lotion huileuse comprenant à titre de support cosmétique, des esters d'acides gras, des huiles et des cires naturelles ou synthétiques.

**7.** Composition selon l'une quelconque des revendications 1 à 4, caractérisée par le fait qu'elle constitue une lotion oléoalcoolique comprenant à titre de support cosmétique des huiles, des cires ou des esters d'acides gras et notamment des triglycérides d'acides gras et des alcools, glycols et/ou polyols inférieurs.

**8.** Composition selon la revendication 4 sous forme d'émulsion ou de dispersion vésiculaire de lipides amphiphiles ioniques ou non-ioniques, caractérisée par le fait qu'elle contient en plus un filtre UV hydrosoluble choisi parmi l'acide 1,4-[di(3-méthylidène 10-camphosulfonique)], l'acide 2-phénylbenzimidazole 5-sulfonique et l'acide 2-hydroxy-4-méthoxybenzophénone 5-sulfonique, salifié ou non et dispersé dans la phase aqueuse.

**9.** Composition selon l'une quelconque des revendications 1 à 8, caractérisée par le fait qu'elle contient en plus des nanopigments d'oxyde métallique dispersés dans la phase grasse et/ou dans la phase aqueuse.

**10.** Composition cosmétique filtrante selon l'une quelconque des revendications 1 à 4, caractérisée par le fait qu'elle constitue un gel oléoalcoolique comprenant à tire de support cosmétique une huile ou une cire naturelle ou synthétique, un alcool ou polyol inférieur et un épaississant.

**11.** Procédé de protection de l'épiderme humain contre les effets des rayons UV de longueurs d'onde comprises entre 320 et 380 nm, caractérisé par le fait qu'il consiste à appliquer sur la peau une quantité efficace d'une composition cosmétique filtrante telle que définie dans l'une quelconque des revendications 1 à 8.

**12.** Procédé de stabilisation du 4-(1,1-diméthyléthyl) 4'-méthoxydibenzoylméthane vis-à-vis du rayonnement UV, caractérisé par le fait que l'on ajoute au moins 0,5% en poids d'un composé de formule (I) définie dans la revendication 1, à 0,5 à 5% en poids de 4-(1,1-diméthyléthyl)-4'-méthoxydibenzoylméthane; le rapport molaire du composé de formule (I) au 4-(1,1-diméthyléthyl)-4'-méthoxydibenzoylméthane étant égal ou supérieur à 0,8.

**13.** Composition cosmétique filtrante photostable pour la protection de l'épiderme humain contre les rayons UV de longueurs d'onde comprises entre 280 et 380 nm, caractérisée par le fait qu'elle comprend dans un support cosmétiquement acceptable de 0,5 à 5% en poids de 4-(1,1-diméthyléthyl) 4'-méthoxy dibenzoylméthane et au moins 0,5% en poids d'un 4-méthoxybenzylidène cyano acétate de formule :

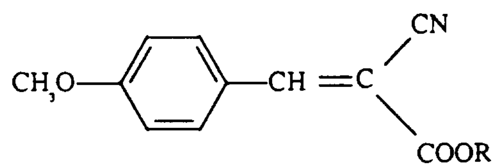

(I)

où R désigne un radical alkyle, linéaire ou ramifié, ayant 6 à 12 atomes de carbone; le rapport molaire du composé de formule (I) au 4-(1,1-diméthyléthyl) 4'-méthoxy dibenzoylméthane étant supérieur ou égal à 0,8 et un ou plusieurs filtres UV-B.

**14.** Composition selon la revendication 13, caractérisée par le fait que les filtres UV-B sont choisis parmi le benzylidène camphre et ses dérivés; les $\beta,\beta$-diphénylacrylates; les dialkylbenzalmalonates; les esters de l'acide salicylique; les esters de l'acide p-amino benzoïque et leurs dérivés, les dérivés de benzophénone, les $\alpha$-cyano $\beta,\beta$-diphénylacrylates d'alkyle.

**15.** Procédé de protection de l'épiderme humain contre les effets des rayons UV de longueurs d'onde comprises entre 280 et 380 nm, caractérisé par le fait qu'il consiste à appliquer sur la peau une quantité efficace d'une composition telle que définie dans la revendication 13 ou 14.

## Claims

1. Photostable cosmetic screening composition for protection of the human epidermis against ultraviolet rays of wavelengths between 320 and 380 nm, characterized in that it comprises, in a cosmetically acceptable carrier, from 0.5 to 5% by weight of 4-(1,1-dimethylethyl)-4'-methoxydibenzoylmethane and at least 0.5% by weight of a (4-methoxybenzylidene)cyanoacetate of formula:

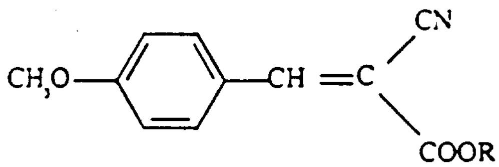

(I)

where R denotes a linear or branched alkyl radical having 6 to 12 carbon atoms; the mole ratio of the compound of formula (I) to 4-(1,1-dimethylethyl)-4'-methoxydibenzoylmethane being greater than or equal to 0.8.

2. Composition according to claim 1, characterized in that the compound of formula (I) is chosen from n-hexyl (4-methoxybenzylidene)cyanoacetate and isononyl (4-methoxybenzylidene)cyanoacetate.

3. Composition according to claim 1 or 2, characterized in that it takes the form of an oily or oleoalcoholic lotion, or the form of a fatty or oleoalcoholic gel, of a solid stick, of an emulsion or of a vesicular dispersion of ionic or nonionic amphiphilic lipids, or is packaged as an aerosol.

4. Composition according to any one of claims 1 to 3, characterized in that it contains, in addition, cosmetic adjuvants chosen from thickeners, emollients, humectants, surfactants, preservatives, antifoams, perfumes, oils, waxes, lower polyols and monohydric alcohols, propellants, dyes and pigments.

5. Composition according to any one of claims 1 to 4, characterized in that it constitutes an emulsion in the form of a cream or milk comprising, as a cosmetic carrier, fatty alcohols, fatty acid esters, and in particular fatty acid triglycerides, fatty acids, lanolin, natural or synthetic oils and waxes and emulsifiers, in the presence of water.

6. Composition according to any one of claims 1 to 4, characterized in that it constitutes an oily lotion comprising, as a cosmetic carrier, fatty acid esters, natural or synthetic oils and waxes.

7. Composition according to any one of claims 1 to 4, characterized in that it constitutes an oleoalcoholic lotion comprising, as a cosmetic carrier, oils, waxes or fatty acid esters, and in particular triglycerides of fatty acids and of lower polyols, alcohols and/or glycols.

8. Composition according to claim 4 in the form of an emulsion or vesicular dispersion of ionic or nonionic amphiphilic lipids, characterized in that it contains, in addition, a water-soluble UV screening agent chosen from 1,4-[di(3-methylidene-10-camphorsulfonic acid)] [sic], 2-phenylbenzimidazole-5-sulfonic acid and 2-hydroxy-4-methoxybenzophenone-5-sulfonic acid, salified or otherwise and dispersed in the aqueous phase.

9. Composition according to any one of claims 1 to 8, characterized in that it contains, in addition, metal oxide nanopigments dispersed in the fatty phase and/or in the aqueous phase.

10. Cosmetic screening composition according to any one of claims 1 to 4, characterized in that it constitutes an oleoalcoholic gel comprising, as a cosmetic carrier, a natural or synthetic oil, a lower polyol or alcohol and a thickener.

11. Process for protecting the human epidermis against the effects of UV rays of wavelengths between 320 and 380 nm, characterized in that it consists in applying an effective amount of a cosmetic screening composition as defined in any one of claims 1 to 8 to the skin.

12. Process for stabilizing 4-(1,1-dimethylethyl)-4'-methoxydibenzoylmethane against UV radiation, characterized in that at least 0.5% by weight of a compound of formula (I) defined in claim 1 is added to 0.5 to 5% by weight of 4-(1,1-dimethylethyl)-4'-methoxydibenzoylmethane; the mole ratio of the compound of formula (I) to 4-(1,1-dimethylethyl)-4'-methoxydibenzoylmethane being equal to or greater than 0.8.

13. Photostable cosmetic screening composition for protection of the human epidermis against UV rays of wavelengths between 280 and 380 nm, characterized in that it comprises, in a cosmetically acceptable carrier, from 0.5 to 5% by weight of 4-(1,1-dimethylethyl)-4'-methoxydibenzoylmethane and at least 0.5% by weight of a (4-methoxybenzylidene)cyanoacetate of formula:

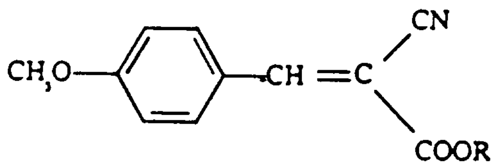

(I)

where R denotes a linear or branched alkyl radical having 6 to 12 carbon atoms; the mole ratio of the compound of formula (I) to 4-(1,1-dimethylethyl)-4'-methoxydibenzoylmethane being greater than or equal to 0.8, and one or more UV-B screening agents.

14. Composition according to claim 13, characterized in that the UV-B screening agents are chosen from benzylidenecamphor and its derivatives; $\beta,\beta$-diphenylacrylates; dialkyl benzalmalonates; salicylic acid esters; p-aminobenzoic acid esters and their derivatives, benzophenone derivatives and alkyl $\alpha$-cyano-$\beta$-$\beta$-diphenylacrylates.

15. Process for protecting the human epidermis against the effects of UV rays of wavelengths between 280 and 380 nm, characterized in that it consists in applying an effective amount of a composition as defined in claim 13 or 14 to the skin.

## Patentansprüche

1. Lichtstabile filternde kosmetische Zusammensetzung zum Schutz der menschlichen Epidermis vor ultravioletten Strahlen von Wellenlängen von 320 bis 380 nm,
dadurch **gekennzeichnet**, daß
sie in einem kosmetisch geeigneten Trägermittel 0,5 bis 5 Gew.% 4-(1,1-Dimethylethyl)-4'-methoxydbenzoylmethan und mindestens 0,5 Gew.% eines 4-Methoxybenzylidencyanoacetats der Formel enthält:

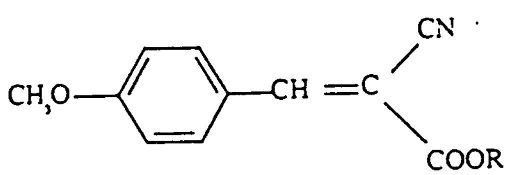

(I)

worin R einen linearen oder verzweigten Alkylrest mit 6 bis 12 Kohlenstoffatomen darstellt, wobei das Molverhältnis der Verbindung der Formel (I) zum 4-(1,1-Dimethylethyl)-4'-methoxydibenzoylmethanmehr als oder 0,8 beträgt.

2. Zusammensetzung gemäß Anspruch 1,
dadurch **gekennzeichnet**, daß
die Verbindung der Formel (I) aus n-Hexyl-4-methoxybenzylidencyanoacetat und Isononyl-4-methoxybenzylidencyanoacetat ausgewählt ist.

3. Zusammensetzung gemäß Anspruch 1 oder 2,
dadurch **gekennzeichnet**, daß
sie in Form einer öligen oder oleoalkoholischen Lotion, in Form eines fetten oder oleoalkoholischen

Gels, eines Feststoffstäbchens, einer Emulsion, bläschenartigen Dispersion aus amphiphilen ionischen oder nicht-ionischen Lipiden vorliegt oder als Aerosol zubereitet ist.

**4.** Zusammensetzung gemäß jedem der Ansprüche 1 bis 3,
dadurch **gekennzeichnet**, daß
sie ausserdem kosmetische Hilfsstoffe enthält, ausgewählt aus Verdickungsmitteln, weichmachenden Mitteln, Feuchtigkeitsmitteln, oberflächenaktiven Mitteln, Konservierungsstoffen, Antischaummitteln, Parfüm-Produkten, Ölen, Wachsen, Monoalkoholen und Niedrigpolyolen, Treibmitteln, Färbemitteln und Pigmenten.

**5.** Zusammensetzung gemäß jedem der Ansprüche 1 bis 4,
dadurch **gekennzeichnet**, daß
sie eine Emulsion in Form einer Creme oder Milch darstellt, die als kosmetischen Träger Fettalkohole, Fettsäureester und insbesondere Triglyceride von Fettsäuren, Fettsäuren, Lanolin, natürliche oder synthetische Öle und Wachse und Emulgatoren, in Gegenwart von Wasser, enthält.

**6.** Zusammensetzung gemäß jedem der Ansprüche 1 bis 4,
dadurch **gekennzeichnet**, daß
sie eine ölige Lotion darstellt, die als kosmetischen Träger Fettsäureester, natürliche oder synthetische Öle und Wachse enthält.

**7.** Zusammensetzung gemäß jedem der Ansprüche 1 bis 4,
dadurch **gekennzeichnet**, daß
sie eine oleoalkoholische Lotion darstellt, die als kosmetischen Träger Öle, Wachse oder Fettsäureester und insbesondere Triglyceride von Fettsäuren und Ester aus Niedrigalkoholen, -glycolen und/oder -polyolen enthält.

**8.** Zusammensetzung gemäß Anspruch 4 in Form einer bläschenartigen Emulsion oder Dispersion aus amphiphilen ionischen oder nicht-ionischen Lipiden,
dadurch **gekennzeichnet**, daß
sie zusätzlich einen wasserlöslichen UV-Filterstoff enthält, ausgewählt aus 1,4-(Di(3-methyliden-10-camphosulfonsäure))benzol, 2-Phenylbenzimidazol-5-sulfonsäure und 2-Hydroxy-4-methoxybenzophenon-5-sulfonsäure enthält, welche in Salzform vorliegen oder nicht und in der wässrigen Phase dispergiert sind.

**9.** Zusammensetzung gemäß jedem der Ansprüche 1 bis 8,
dadurch **gekennzeichnet**, daß
sie zusätzlich Nanopigmente aus Metalloxid enthält, welche in der fetten und/oder wässrigen Phase dispergiert sind.

**10.** Filternde kosmetische Zusammensetzung gemäß jedem der Ansprüche 1 bis 4,
dadurch **gekennzeichnet,** daß
sie ein oleoalkoholisches Gel darstellt, das als kosmetischen Träger ein natürliches oder synthetisches Öl oder Wachs, einen Niedrigalkohol oder ein Niedrigpolyol und ein Verdickungsmittel enthält.

**11.** Verfahren zum Schutz der menschlichen Epidermis vor den Wirkungen von UV-Strahlen einer Wellenlänge von 320 bis 380 nm,
dadurch **gekennzeichnet,** daß
das Verfahren darauf beruht, daß man auf die Haut eine wirksame Menge einer in jedem der Ansprüche 1 bis 8 definierten, filternden kosmetischen Zusammensetzung aufträgt.

**12.** Verfahren zur Stabilisierung des 4-(1,1-Dimethylethyl)-4'-methoxydibenzoylmethans gegenüber der UV-Strahlung,
dadurch **gekennzeichnet**, daß
man mindestens 0,5 Gew.% einer in Anspruch 1 definierten Verbindung der Formel (I) zu 0,5 bis 5 Gew.% 4-(1,1-Dimethylethyl)-4'-methoxydibenzoylmethan gibt, wobei das Molverhältnis der Verbindung der Formel (I) zum 4-(1,1-Dimethylethyl)-4'-methoxydibenzoylmethan 0,8 oder mehr beträgt.

**13.** Lichtstabile filternde kosmetische Zusammensetzung zum Schutz der menschlichen Epidermis vor UV-Strahlen von Wellenlängen von 280 bis 380 nm,
dadurch **gekennzeichnet**, daß
sie in einem kosmetisch geeigneten Träger 0,5 bis 5 Gew.% 4-(1,1-Dimethylethyl)-4'-methoxydibenzoylmethan und mindestens 0,5 Gew.% eines 4-Methoxybenzylidencyanoacetats der Formel:

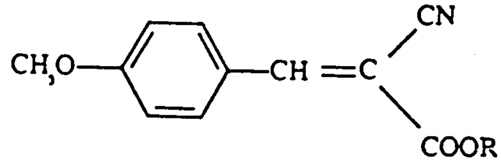

(I)

worin R einen linearen oder verzweigten Alkylrest mit 6 bis 12 Kohlenstoffatomen darstellt, wobei das Molverhältnis der Verbindung der Formel (I) zum 4-(1,1-Dimethylethyl)-4'-methoxydibenzoylmethanmehr als oder 0,8 beträgt, und einen oder mehrere UV-B-Filterstoffe enthält.

**14.** Zusammensetzung gemäß Anspruch 13,
dadurch **gekennzeichnet**, daß
die UV-B-Filterstoffe aus Benzylidenkampfer und seinen Derivaten, $\beta,\beta$-Diphenylacrylaten, Dialkylbenzalmalonaten, Salicylsäureestern, p-Aminobenzoesäureestern und seinen Derivaten, Benzophenonderivaten, Alkyl-$\alpha$-cyano-$\beta,\beta$-diphenylacrylaten ausgewählt sind.

**15.** Verfahren zum Schutz der menschlichen Epidermis vor den Wirkungen von UV-Strahlen einer Wellenlänge von 280 bis 380 nm,
dadurch **gekennzeichnet,** daß
das Verfahren darauf beruht, daß man auf die Haut eine wirksame Menge einer in Anspruch 13 oder 14 definierten Zusammensetzung aufträgt.